# EUROPEAN PATENT APPLICATION

(11) **EP 1 078 630 A1**
(43) Date of publication of application: **28.02.2001**
(21) Application number: 99402143.4
(22) Date of filing: 27.08.1999
(51) Int. Cl.: A61K 31/135, A61K 31/435, A61K 31/495

(54) **Use of sigma receptor agonists for the treatment of depression**

(71) Applicant: WARNER-LAMBERT COMPANY, Morris Plains New Jersey 07950 (US)
(72) Inventor: Maurice, Tangui, 34980 Montferrier-sur-Lez (FR); Roman, François, 94400 Vitry sur Seine (FR)
(74) Representative: Hirsch, Marc-Roger

(57) **Abstract**

Method for the prevention or treatment of depression comprising administering to a steroid-depleted patient suffering therefrom and in need of treatment an effective amount of a sigma 1 receptor agonist.

## Description

### BACKGROUND OF THE INVENTION

Depression is a serious illness associated with a high morbidity resulting from physical disorders and an increased mortality resulting from accidents and suicide. Major depression recurs in about half the patients. Thus depressed patients may be treated with antidepressants continuously for years. There are, however, important drawbacks to the use of available drugs. Twenty-five to 35 % of patients show only minimal improvement, and side effects and toxicity can occur: tricyclic antidepressants (TCAs) are especially bothersome in elderly patients, with postural hypotension being a particularly severe problem; selective serotonin reuptake inhibitors (SSRIs) may induce nausea, vomiting, diarrhea, headache and sexual dysfunctions. Severe drug interactions can also result from the co-administration of monoamine oxidase inhibitors and either TCAs or SSRIs.
As a consequence, there is a need for antidepressant drugs that would be active at doses intended to be as low as possible in order to minimize the risk of side effects and drug interactions, especially in elderly patients, often exposed to several drugs concurrently.
Sigma 1 (σ₁) receptor ligands show clear antidepressant effects in several animal models: the selective σ₁ receptor agonists (+)-pentazocine, (+)-SKF-10,047, igmesine, OPC14523 , DTG or SA4503 reduce the immobility time in the forced swim test or are active in the tail suspension test (Ukai et al. 1998, Matsuno et al., 1996, Tottori et al. 1997, Kinsora et al. 1998).
United States patent 5,034,419 describes N-cycloalkylalkylamines, which are sigma 1 receptor agonists.

### SUMMARY OF THE INVENTION

It has now been found that the antidepressant effect of selective σ₁ receptor agonists is affected by the level of endogenous steroids. More particularly, σ₁ receptor agonists, in particular the compounds described in US 5,034,419, are useful for the prevention or treatment of depression, especially in steroid-depleted patients.

This invention therefore provides a method for preventing or treating depression, said method comprising administering to a steroid-depleted patient in need of such treatment an effective amount of a sigma 1 receptor agonist.
Preferred sigma 1 receptor agonists include those of Formula I, described in US 5,034,419, 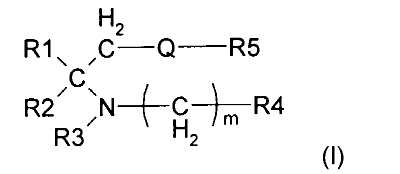 wherein:
R1 is phenyl optionally mono-, di- or trisubstituted by halogen, lower alkyl, lower haloalkyl or lower alkoxy,
R2 is lower alkyl,
R3 is hydrogen or lower alkyl,
R4 is cycloalkyl -CH(CH₂)ₙ wherein n has a value of 2, 3, 4 or 5 and wherein a carbon atom of R4 optionally carries a radical Rₓ wherein Rₓ is lower alkyl or phenyl,
R5 is phenyl optionally mono-, di- or trisubstituted by halogen or lower alkoxy,
Q represents an ethylene-1,2-yl group -CH=CH- or a cyclopropane-1,2-diyl group 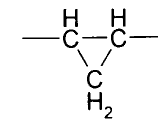 and
m has a value of 1 or 2,
the pharmaceutically acceptable salts thereof and the optically active forms thereof.

This invention also concerns the use of a sigma receptor agonist for the preparation of a medicament useful for preventing or treating depression in steroid-depleted patients.

### BRIEF DESCRIPTION OF FIGURES

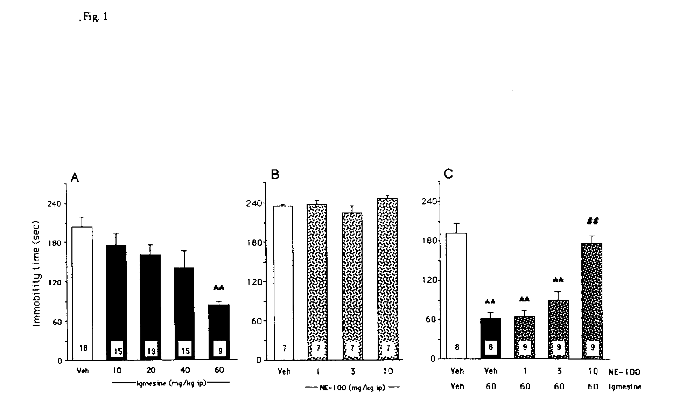

Figure 1 shows the antidepressant effect of igmesine in the forced swim test in Swiss mice **(A)**, lack of effect of NE-100 **(B)**, and antagonism by NE-100 of the igmesine-induced effect **(C)**.

Drugs were injected i.p. 30 min before the session on day 2. NE-100 was administered i.p. 15 min before igmesine, which was given 30 min before the session on day 2.
The duration of immobility was recorded during the last 5 min over a 6-min session.
Values are expressed as mean ± S.E.M. of the number of animals indicated inside each column.
* P < 0.05, ** P < 0.01 vs. the Vehicule-treated group; ^{##} P < 0.01 vs. the igmesine-treated group (Dunnett's test).

Figure 2 shows the antidepressant effect in the forced swim test in Swiss mice of desipramine **(A)** and fluoxetine **(B)**.
Drugs were injected i.p. 30 min before the session on day 2. Values are expressed as mean ± S.E.M. of the number of animals indicated inside each column.
** P < 0.01 vs the Vehicle-treated group (Dunnett's test).

Figure 3 shows the antidepressant effect of neurosteroids in the forced swim test in Swiss mice: dose-response effect of DHEAS **(A)**, PREGS **(B)**, PROG **(C)**, and antagonism by PROG of the DHEAS-induced effect **(D)**.
The steroids were injected i.p. 30 min before the session on day 2. PROG was administered i.p. 15 min before DHEAS, which was given 30 min before the test. Values are expressed as mean ± S.E.M. of the number of animals indicated inside each column. * P < 0.05, ** P < 0.01 vs. the Veh-treated group; ^{##} P < 0.01 vs. the DHEAS-treated group (Dunnett's test).

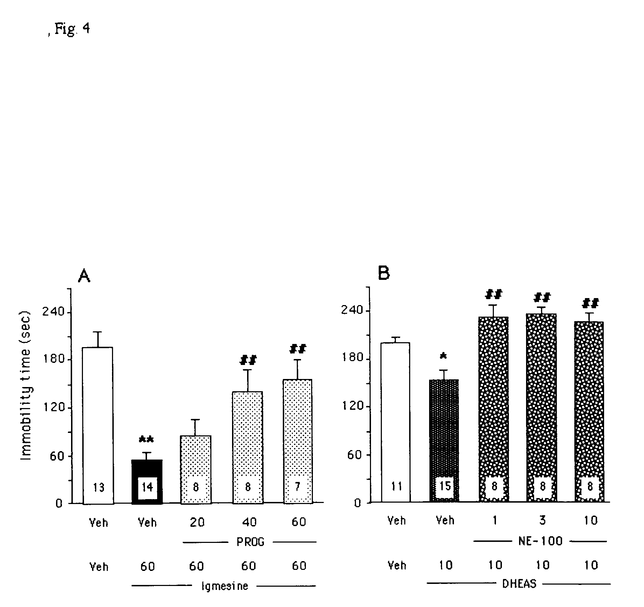

Figure 4 shows the antagonism of the antidepressant effect of igmesine by PROG **(A)** and of the DHEAS-induced effect by NE-100 **(B)**.
PROG or NE-100 was administered i.p. 15 min before igmesine or DHEAS, respectively, which was given 30 min before the test. Values are expressed as mean ± S.E.M. of the number of animals indicated inside each column. * P < 0.05, ** P < 0.01 vs. the Veh-treated group; ^{##} P < 0.01 vs. the igmesine- or DHEAS-treated group (Dunnett's test).

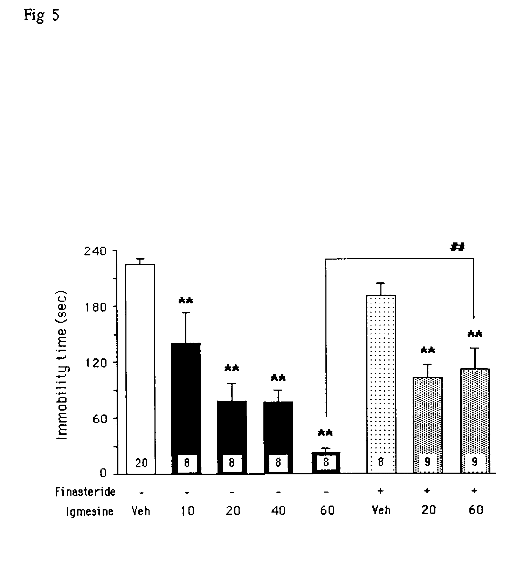

Figure 5 shows the antidepressant effect of igmesine in the forced swim test in AdX/CX Swiss mice. Half of experimental groups were treated with finasteride (25 mg/kg, s.c.) 14 and 2 hr before the session on day 2. The σ₁ ligands were injected i.p. 30 min before the session. Values are expressed as mean ± S.E.M. of the number of animals indicated inside each column.
** P < 0.01 vs. the Veh-treated group; ^{##} P < 0.01 vs. the igmesine- or PRE-084-treated group (Dunnett's test).

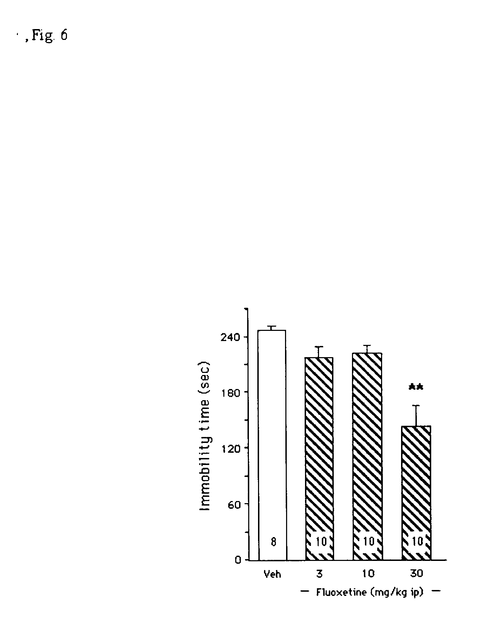

Figure 6 shows the antidepressant effect of fluoxetine in the forced swim test in AdX/CX Swiss mice. The drug was injected i.p. 30 min before the session on day 2. Values are expressed as mean ± S.E.M. of the number of animals indicated inside each column.
** P < 0.05, ** P < 0.01 vs. the Veh-treated group (Dunnett's test).

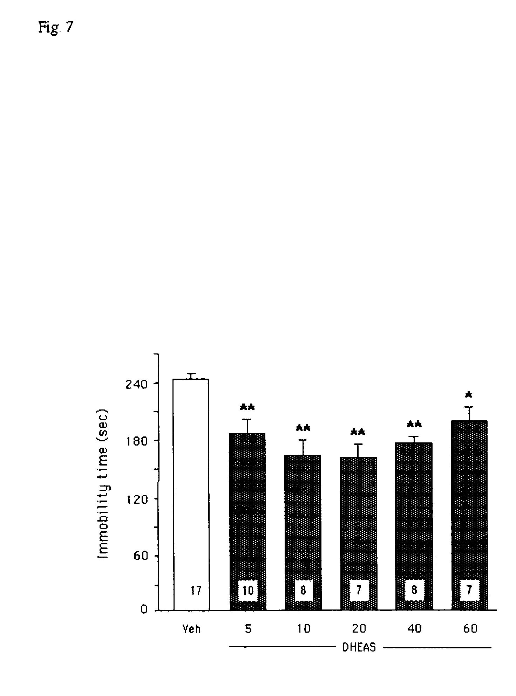

Figure 7 shows the antidepressant effect of DHEAS in the forced swim test in AdX/CX Swiss mice. Half of experimental groups were treated with finasteride (25 mg/kg, s.c.) 14 and 2 hr before the session on day 2. The steroid was injected i.p. 30 min before the session. Values are expressed as mean ± S.E.M. of the number of animals indicated inside each column.
** P < 0.05, ** P < 0.01 vs. the Vehicle-treated group (Dunnett's test).

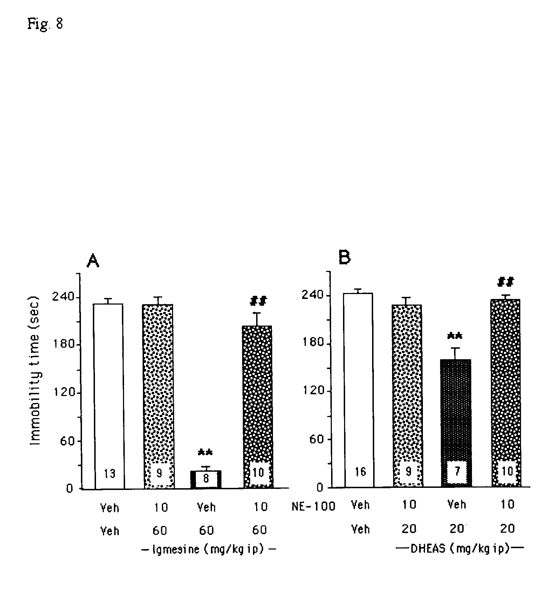

Figure 8 shows the antagonism of the antidepressant effect of igmesine **(A)** or DHEAS **(B)** by NE-100 in AdX/CX Swiss mice. NE-100 was administered i.p. 15 min before igmesine or DHEAS, which was given 30 min before the test. Values are expressed as mean ± S.E.M. of the number of animals indicated inside each column. ** P < 0.01 vs. the Veh-treated group; ^{##} P < 0.01 vs. the igmesine- or DHEAS-treated group (Dunnett's test).

### DETAILED DESCRIPTION

Neurosteroids are steroids that are synthesized in the brain from sterol precursors (Baulieu, 1981). Neurosteroids include:
- progesterone (PROG),
- 5α-pregnane-3β-ol-20-one (allopregnanolone),
- pregnenolone (PREG),
- dehydroepiandrosterone (DHEA), and
- PREG and DHEA sulfate esters (PREGS and DHEAS respectively).
Apart from their well-known effects on the control of gene expression, neurosteroids modulate several neurotransmission systems, in an excitatory or inhibitory way (Rupprecht et al. 1996).
PREGS and DHEAS act as excitatory neurosteroids, since they antagonize the activation of γ-aminobutyric acid type A (GABA_{A}) receptors, whereas they potentiate the activation of the N-methyl-D-aspartate (NMDA)-type of glutamatergic receptors.
Other neurosteroids including PROG and allopregnanolone act as inhibitory neurosteroids, being very potent agonists of GABA_{A} receptors with affinities comparable to those of benzodiazepines. Neurosteroids are involved in several physiopathological events, such as response to stress, depression, anxiety, sleep, epilepsy and memory formation (for a review, see Schumacher et al. 1997).
The interaction between neurosteroids and the σ₁ receptor have been uncovered in binding studies (Maurice et al. 1996; Su et al. 1988; Yamada et al. 1994), then reported in physiological studies regarding several neuronal responses (Bergeron et al. 1994; Debonnel et al. 1996; Monnet et al. 1995). However the exact nature of this interaction, and how it can be used to influence disease states, still remained to be determined.
The inventors have now shown that in steroid-depleted mammals, the antidepressant effect of σ₁ receptor agonists is optimized when endogenous steroid levels are low.

The compounds used in the invention are σ₁ receptor agonists.

Preferred compounds are those of Formula I 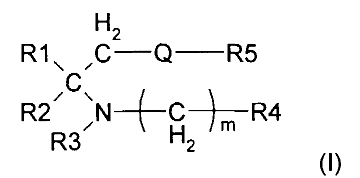 wherein
R1 is phenyl optionally mono-, di- or trisubstituted by halogen, lower alkyl, lower haloalkyl or lower alkoxy,
R2 is lower alkyl,
R3 is hydrogen or lower alkyl,
R4 is cycloalkyl -CH(CH₂)ₙ wherein n has a value of 2, 3, 4 or 5 and wherein a carbon atom of R4 optionally carries a radical Rₓ wherein Rₓ is lower alkyl or phenyl,
R5 is phenyl optionally mono-, di- or trisubstituted by halogen or lower alkoxy,
Q represents an ethylene-1,2-yl group -CH=CH- or a cyclopropane-1,2-diyl group 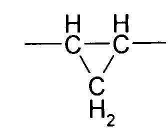 and
m has a value of 1 or 2,
the pharmaceutically acceptable salts thereof and the optically active forms thereof.
Other preferred compounds are those of Formula I wherein
Other preferred compounds are those of Formula I wherein
The lower alkyl groups of the compounds used in the invention include straight, branched, or cyclic carbon chains of from 1 to 5 carbon atoms except where specifically stated otherwise.
Representative groups are methyl, ethyl, isopropyl, *n*-propyl, *n*-butyl, *iso*-butyl, *sec*-butyl, *tert*-butyl, *n*-pentyl, 1-methylbutyl, 2,2-dimethylpropyl, and the like.
By haloalkyl and lower alkoxy, there are understood, preferably, the trifluoromethyl and methoxy radicals respectively.
The term "halogen" is intended to include fluorine, bromine and ,preferably, chlorine.

A more preferred compound is (E)-(+)-N-(cyclopropylmethyl)-α-ethyl-N-methyl-α-(3-phenyl-2-propenyl)benzenemethanamine hydrochloride, also known as igmesine hydrochloride (INN).

The compounds used in the present invention can have multiple chiral centers in the above Formula I depending on their structure. In particular, the compounds used in the present invention may exist as diastereomers, mixtures of diastereomers, or as the mixed or the individual optical enantiomers. The present invention contemplates use of all such forms of the compounds.
The compounds utilized in the invention include solvates, hydrates, pharmaceutically acceptable salts, and polymorphs (different crystalline lattice descriptors) of the compounds of Formula I.
Where it is appropriate to form a salt, the pharmaceutically acceptable salts include acetate, benzenesulfonate, benzoate, bicarbonate, bitartrate, bromide, calcium acetate, camsylate, carbonate, chloride, citrate, dihydrochloride, edetate, edisylate, estolate, esylate, fumarate, gluceptate, gluconate, glutamate, glycoloylarsanilate, hexylresorcinate, hydrabamine, hydrobromide, hydrochloride, hydroxynaphthoate, iodide, isethionate, lactate, lactobionate, malate, maleate, mandelate, mesylate, methylbromide, methylnitrate, methylsulfate, mucate, napsylate, nitrate, pamoate (embonate), pantothenate, phosphate/diphosphate, polygalacturonate, salicylate, stearate, subacetate, succinate, sulfate, tannate, tartrate, theoclate, triethiodide, benzathine, chloroprocaine, choline, diethanolamine, ethylenediamine, meglumine, procaine, aluminum, calcium, lithium, magnesium, potassium, sodium, and zinc. (See also "Pharmaceutical salts" by Berge S.M. *et al.* (1997) J. Pharm. Sci. **66**: 1-19, which is incorporated herein by reference.)
Use of a prodrug of a compound of the invention, such as would occur to one skilled in the art (see Bundgaard, *et al.,* Acta Pharm. Suec., 1987; **24**: 233-246), is also contemplated.
The terms "patient" and "subject" is intended to include a mammal, especially a human. It should also be noted that the method of the present invention can be used in patients presenting either low levels of neurosteroids, low levels of circulating steroids, or both.

The methods of this invention are carried out by administering to a mammal an effective amount of a σ₁ receptor agonist, preferably a compound of Formula I, or a pharmaceutically acceptable salt thereof, to treat the disorders hereinbefore described.
Such effective amount will generally be from about 1 to about 400 mg per day. Preferred doses will be from about 10 mg to about 100 mg per day for an adult of 70 kg. More generally, the dose to be administered to steroid-depleted subjects will be about 5 to 50 % and preferably about 10 to 40 % of the average dose used for non steroid-depleted subjects.
In a further aspect of the present invention, there is provided a pharmaceutical composition for the treatment or prevention of depression comprising a sigma 1 receptor agonist of Formula I, together with at least one pharmaceutically acceptable carrier or excipient. For preparing pharmaceutical compositions from the compounds used in this invention, inert, pharmaceutically acceptable carriers can be either solid or liquid. Solid form preparations include powders, tablets, dispersible granules, capsules, cachets, and suppositories. A solid carrier can be one or more substances which may also act as diluents, flavoring agents, solubilizers, lubricants, suspending agents, binders, or tablet disintegrating agents; it can also be an encapsulating material. In powders, the carrier is a finely divided solid which is in a mixture with the finely divided active component. In tablets, the active component is mixed with the carrier having the necessary binding properties in suitable proportions and compacted in the shape and size desired. For preparing suppository preparations, a low-melting wax such as a mixture of fatty acid glycerides and cocoa butter is first melted and the active ingredient is dispersed therein by, for example, stirring. The molten homogeneous mixture is then poured into convenient sized molds and allowed to cool and solidify. The powders and tablets preferably contain 5% to about 70% of the active component. Suitable carriers are magnesium carbonate, magnesium stearate, talc, lactose, sugar, pectin, dextrin, starch, tragacanth, methyl cellulose, sodium carboxymethyl cellulose, a low-melting wax, cocoa butter, and the like.
The term "preparation" is intended to include the formulation of the active component with encapsulating material as a carrier providing a capsule in which the active component (with or without other carriers) is surrounded by a carrier which is thus in association with it. Similarly, cachets are included.
Tablets, powders, cachets, and capsules can be used as solid dosage forms suitable for oral administration.
Liquid form preparations include solutions, suspensions, and emulsions.
Sterile water or water-propylene glycol solutions of the active compounds may be mentioned as an example of liquid preparations suitable for parenteral administration. Liquid preparations can also be formulated in solution in aqueous polyethylene glycol solution.
Aqueous solutions for oral administration can be prepared by dissolving the active component in water and adding suitable colorants, flavoring agents, stabilizers, and thickening agents as desired. Aqueous suspensions for oral use can be made by dispersing the finely divided active component in water together with a viscous material such as natural synthetic gums, resins, methyl cellulose, sodium carboxymethyl cellulose, and other suspending agents known to the pharmaceutical formulation art.
Preferably the pharmaceutical preparation is in unit dosage form. In such form, the preparation is divided into unit doses containing appropriate quantities of the active component. The unit dosage form can be a packaged preparation, the package containing discrete quantities of the preparation, for example, packeted tablets, capsules, and powders in vials or ampoules. The unit dosage form can also be a capsule, cachet, or tablet itself, or it can be the appropriate number of any of these packaged forms.

### EXAMPLES

The compounds of the instant invention are useful in the treatment of depression in steroid-depleted patients as demonstrated by means of the following pharmacological procedures.

Using the forced swim test in mice, through several endocrine manipulations ―adrenalectomy/castration and inhibition of the 5α-reductase responsible for the formation of progesterone― it is demonstrated that endogenous steroidal levels have a major influence on the antidepressant effect of σ₁ receptor agonists.

### Animals

Male Swiss OF1 mice (Breeding center of the Faculty of Pharmacy, Montpellier, France), aged 5-6 weeks and weighing 30 ± 2 g are used throughout the study. Animals are housed in plastic cages in groups. They have free access to laboratory chow and water, except during behavioral experiments, and they are kept in a regulated environment (23 ± 1°C, 40-60% humidity) under a 12-hr light/dark cycle (light on at 7:00 a.m.). Experiments are carried out between 9:00 a.m. and 6:00 p.m., in a soundproof and air-regulated experimental room, to which mice are habituated at least 30 min before each experiment.

### Drugs

PROG and finasteride are suspended in sesame oil ; other drugs are solubilized in distilled water or saline solution. Drugs are injected subcutaneously (s.c.) or intraperitoneally (i.p.), in a volume of 100 µl per 20 g of body weight.

### Forced swim test

Each mouse is placed individually in a glass cylinder (diameter 12 cm, height 24 cm) filled with water at a height of 12 cm. Water temperature is maintained at 22-23°C. The animal is forced to swim for 15 min on the first day. Animals are then allowed to return to their home cage. On the second day, each mouse is placed again into the water and forced to swim for 6 min. The session is videotaped and the duration of immobility during the last 5 min is measured. The mouse is considered as immobile when it stops struggling and moves only to remain floating in the water, keeping its head above water. Drugs are administered 30 min before the session on the second day. Finasteride is administered twice, 14 and 2 hr before the session on day 2.

### Adrenalectomy (AdX) / castration (CX)

About one week after habituation to the animal facilities, animals are anaesthetized with sodium pentobarbital 2%, 100 µl/30 g body weight i.p. Both adrenal glands are removed, through incisions in the back of the animal, just below the breast ribs. The skin is sutured. Then, both testes are ligatured and cut, through an incision in the scrotum. Animals received an injection of gentamycine 10 mg/kg i.p. and recovered within few hours from surgery. After surgery, drinking tap water is replaced by saccharose 1%, NaCI 0.9% solution. Animals are used for behavioral experiments six days after surgery.

### Statistical analysis

Results are expressed as means ± S.E.M. Data are analyzed using the Dunnett's multiple comparisons test after an analyzis of variance (ANOVA, F values). The criterion for statistical significance is P < 0.05.

### Effects of σ₁ receptor agonists on the immobility time in the forced swim test

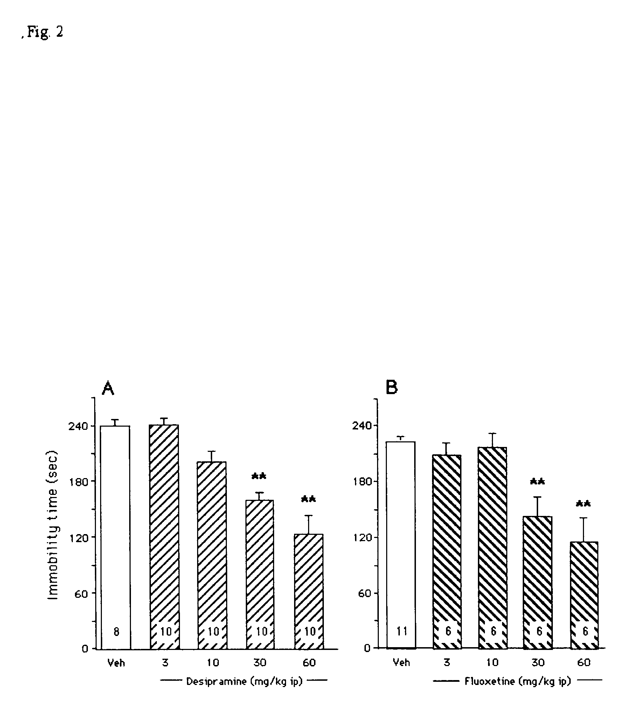

Mice rapidly develop a marked behavioral despair during the forced swim, with an immobility time of 180 ± 4 s (n = 18) on the first day. This immobility time significantly increases up to 227 ± 5 s on the second day (t = 7.92, P < 0.01, paired *t*-test).
The selective σ₁ receptor agonist igmesine significantly shortens the immobility time in the forced swim test at the dose of 60 mg/kg i.p. (F_{(4,75)} = 4.43, P < 0.01; Fig. 1A).
Similarly, the reference σ₁ receptor agonist (+)-SKF-10,047 decreases the immobility time at the dose of 30 mg/kg (Fig. 1B).
The selective σ₁ receptor antagonist NE-100 (Ukai et al., 1998), tested in the 1-10 mg/kg i.p. dose-range, does not affect the immobility time by itself (F_{(3,27)} = 1.52, P > 0.05; Fig. 1C).
However, pre-administration of NE-100, at 10 mg/kg, completely antagonizes the reduction of immobility time induced by igmesine (F_{(4,42)} = 24.75, P < 0.001; Fig. 1D), confirming that the drug acts through the involvement of the σ₁ receptor.
The effects of the σ₁ receptor agonists are compared to those exhibited by the antidepressant drugs desipramine and fluoxetine (Fig. 2). Both drugs shortened the immobility time, at the doses of 30 and 60 mg/kg i.p. (desipramine: F_{(4,47)} = 17.76, P < 0.001; Fig. 2A; fluoxetine: F_{(4,39)} = 8.00,P < 0.001; Fig. 2B).

### Effects of neurosteroids on the immobility time in the forced swim test

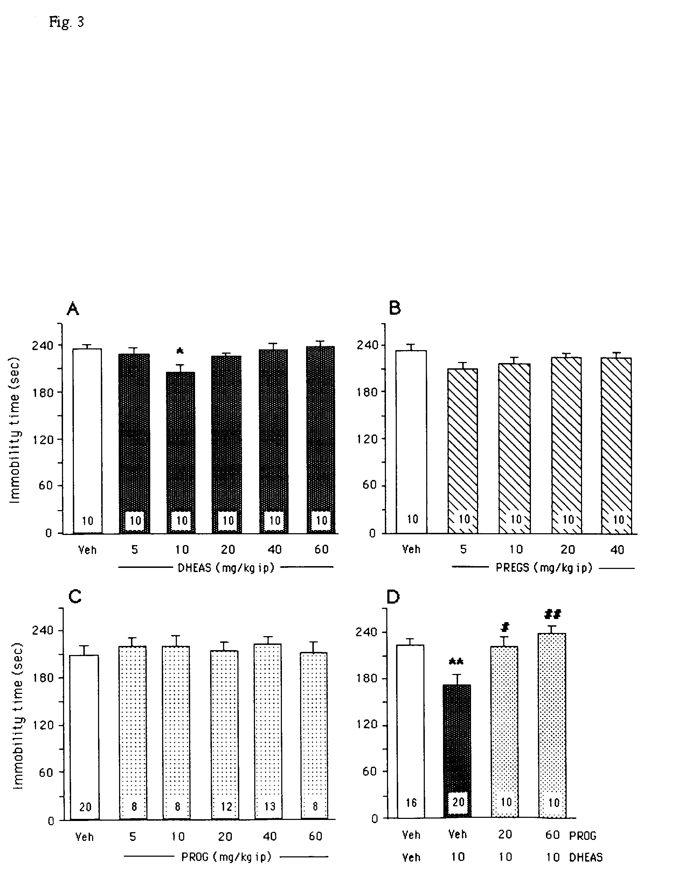

DHEAS, administered in the 5-60 mg/kg dose range, slightly but significantly shortens the immobility time, at the 10 mg/kg dose (F_{(5,89)} = 2.80, P < 0.05; Fig. 3A). At lower or higher dosages, the steroid is devoid of effect.
PREGS fails to affect the immobility time, in the 5-40 mg/kg dose range (F_{(4,74)} = 1.74, P > 0.05; Fig. 3B).
PROG does not affect the immobility time by itself, in the 5-60 mg/kg dose range (F_{(5,68)} = 0.21, P > 0.05; Fig. 3C). However, pre-administration of PROG (20-60 mg/kg) before the DHEAS (10 mg/kg)-treatment leads to a significant antagonism of its effect as shown in Fig. 3D (F_{(3,55)}=6.46, P < 0.001).
Interestingly, a crossed pharmacology is observed between the effects induced by the σ₁ receptor ligands and the steroids. First, the pre-administration of PROG fully blocks the igmesine-induced reduction of immobility (F_{(6,75)} = 15.75, P < 0.001; Fig. 4A). Second, the selective σ₁ receptor antagonist NE-100 also prevents the DHEAS-induced diminution of immobility time (F_{(4,49)}=9.68, P < 0.001; Fig. 4B).

*Effects of σ*_{*1*} *receptor ligands on the immobility time in the forced swim test in AdX/CX animals* AdX/CX animals show an immobility time of 178 ± 12 s (n = 12) on the first day, which significantly increases up to 242 ± 5 s on the second day (t = 4.26, P < 0.01, paired *t*-test). These values do not differ from those in control animals (P > 0.05 on each day). Igmesine shortens significantly the immobility time in AdX/CX animals, in a dose-dependent manner at 20 and 60 mg/kg (F_{(5,49)} = 18.70, P < 0.001; Fig. 5), thus in a more efficient manner as compared to non-operated animals (Fig. 1 A). Finasteride pretreatment, which leads to an increase in the endogenous levels of PROG, does not affect the immobility time exhibited by vehicle-treated AdX/CX mice, but significantly alters the diminution induced by igmesine, at 60 mg/kg (Fig. 5).

### Effects of neurosteroids on the immobility time in the forced swim test in AdX/CX animals

In AdX/CX animals, DHEAS significantly shortens the immobility time, as shown in Fig. 7. The DHEAS treatment leads to significant diminution of the immobility time at all the doses examined, within the 5-60 mg/kg dose-range (F_{(5,56)} = 10.86, P < 0.001; Fig. 7) , thus in a more efficient manner as compared to non-operated animals (Fig. 3A).
The facilitation of the effect observed in AdX/CX mice can be clearly linked to a facilitation of the σ₁ receptor-mediated effect, as confirmed using a pretreatment with NE-100 (Fig. 8).
Indeed, NE-100 fully antagonizes both the antidepressant effect of igmesine in AdX/CX mice (F_{(3,39)} = 71.40, P < 0.001; Fig. 8A) and that of DHEAS (F(3,41) = 19.48, P < 0.001; Fig. 8B).

The effect of igmesine is enhanced in AdX/CX animals, as compared to non-operated mice. Igmesine is active at 10 mg/kg whereas in intact animals the effect occurs only at 60 mg/kg.

Such observations show that circulating steroids exert a tonic modulatory effect on the σ₁ receptor-mediated antidepressant effect. They indicate that endogenous steroids tonically interfere with the antidepressant activity of igmesine, and that the efficacy of igmesine is optimized when endogenous steroidal levels are low.
Furthermore, selective σ₁ receptor antagonist NE-100 antagonized the igmesine-mediated effect, clearly demonstrating the involvement of the σ₁ receptor.

As a consequence, patients with lower levels of steroids, such as elderly patients (particularly patients of age 50 and over, more particularly of age 55 and over) and post-menopausal women, are particularly sensitive to treatment with a sigma 1 receptor agonist.

Interestingly, the effect of the antidepressant drugs fluoxetine and desipramine failed to be affected by the removal of circulating steroids, clearly indicating that the mechanism of the antidepressant effect mediated by σ₁ receptors does not involve an indirect inhibition of the monoamine transporters.
Therefore, sigma 1 receptor agonists, which have a different mechanism of action from that of serotonin reuptake inhibitors (or other monoamine transporter inhibitors), may be of benefit in depressed patients not responsive to these monoamine transporter inhibitors.

### References

Baulieu EE (1981) Steroid hormones in the brain: Several mechanisms? In: Fuxe K, Gustafson JA, Wettenberg L (eds) Steroid Hormone Regulation of the Brain. Pergamon Press, Oxford, pp 3-14
Bergeron R, De Montigny C, Debonnel G (1994) Progesterone suppresses the potentiation of the NMDA response induced by selective sigma ligands. Soc Neurosci Abstr 20: 748
Debonnel G, Bergeron R, de Montigny C (1996) Potentiation by dehydroepiandrosterone of the neuronal response to N- methyl-D-aspartate in the CA3 region of the rat dorsal hippocampus: an effect mediated via s (sigma) receptors. J Endocrinol 150 Suppl: S33-42
Kinsora JJ, Corbin AE, Snyder BJ, Wiley JN, Meltzer LT, Heffner TG (1998)
   Effects of igmesine in preclinical antidepressant tests. Soc Neurosci Abstr 24: 744
   Matsuno K, Kobayashi T, Tanaka MK, Mita S (1996) s1 receptor subtype is involved in the relief of behavioral despair in the mouse forced swimming test. Eur J Pharmacol 312: 267-71
Maurice T, Roman FJ, Privat A (1996) Modulation by neurosteroids of the in vivo (+)-[3H]SKF-10,047 binding to s1 receptors in the mouse forebrain. J Neurosci Res 46: 734-43
Monnet FP, Mahe V, Robel P, Baulieu EE (1995) Neurosteroids, via sigma receptors, modulate the [3H]norepinephrine release evoked by N-methyl-D-aspartate in the rat hippocampus. Proc Natl Acad Sci USA 92: 3774-8
Rupprecht R, Hauser CA, Trapp T, Holsboer F (1996) Neurosteroids: molecular mechanisms of action and psychopharmacological significance. J Steroid Biochem Mol Biol 56: 163-8
Schumacher M, Guennoun R, Robel P, Baulieu EE (1997) Neurosteroids in the Hippocampus: Neuronal Plasticity and Memory. Stress 2: 65-78
Su TP, London ED, Jaffe JH (1988) Steroid binding at sigma receptors suggests a link between endocrine, nervous, and immune systems. Science 240: 219-21
Tottori K, Kikuchi T, Uwahodo Y, Yamada S, Oshiro Y, Koga N (1997) Antidepressant effect of OPC-14523 in the forced swimming test. Jpn J Pharmacol 73SI: 59P
Ukai M, Maeda H, Nanya Y, Kameyama T, Matsuno K (1998) Beneficial effects of acute and repeated administrations of sigma receptor agonists on behavioral despair in mice exposed to tail suspension. Pharmacol Biochem Behav 61: 247-52
Yamada M, Nishigami T, Nakasho K, Nishimoto Y, Miyaji H (1994) Relationship between sigma-like site and progesterone-binding site of adult male rat liver microsomes. Hepatology 20: 1271-80

## Claims

1. A method for treating depression comprising administering to a steroid-depleted patient suffering therefrom and in need of treatment an effective amount of a sigma 1 receptor agonist.

2. A method according to Claim 1 wherein the sigma 1 receptor agonist is a compound of Formula I 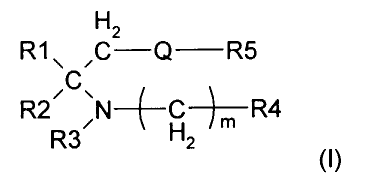 wherein:
R1 is phenyl optionally mono-, di- or trisubstituted by halogen, lower alkyl, lower haloalkyl or lower alkoxy,
R2 is lower alkyl,
R3 is hydrogen or lower alkyl,
R4 is cycloalkyl -CH(CH₂)ₙ wherein n has a value of 2, 3, 4 or 5 and wherein a carbon atom of R4 optionally carries a radical Rₓ wherein Rₓ is lower alkyl or phenyl,
R5 is phenyl optionally mono-, di- or trisubstituted by halogen or lower alkoxy,
Q represents an ethylene-1,2-yl group -CH=CH- or a cyclopropane-1,2-diyl group 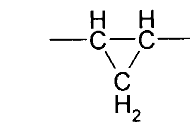 and
m has a value of 1 or 2,
the pharmaceutically acceptable salts thereof and the optically active forms thereof.

3. A method according to Claim 1 wherein the sigma 1 receptor agonist is igmesine.

4. A method according to Claim 1 wherein the steroid-depleted patient is an elderly patient.

5. A method according to Claim 4 wherein the elderly patient is a human of age 50 or older.

6. A method according to Claim 1 wherein the steroid-depleted patient is a post-menopausal woman.

7. A method according to Claim 1 wherein the steroid-depleted patient is not responsive to monoamine transporter inhibitors.

8. A method according to Claim 1 wherein the steroid-depleted patient has depleted neurosteroid levels.

9. A method according to Claim 1 wherein the steroid-depleted patient has depleted circulating steroid levels.

10. A method for preventing depression in a steroid-depleted patient comprising administering to a subject at risk thereof and in need of prophylaxis an effective amount of a sigma 1 receptor agonist.

11. The use of a sigma 1 receptor agonist for the preparation of a medicament useful for preventing or treating depression in steroid-depleted patients and in patients not responsive to monoamine transporter inhibitors.
